# EUROPEAN PATENT SPECIFICATION

(11) **EP 3 394 283 B1**
(45) Date of publication and mention of the grant of the patent: **14.07.2021**
(21) Application number: 16836035.2
(22) Date of filing: 30.08.2016
(51) Int. Cl.: B01L 3/02, G01N 33/487

(54) **SYSTEMS AND METHODS ENABLING PATCH-CLAMP RE-USE**
SYSTEME UND VERFAHREN ZUR ERMÖGLICHUNG DER PATCH-CLAMP-WIEDERVERWENDUNG
SYSTÈMES ET PROCÉDÉS PERMETTANT LA RÉUTILISATION D'UN DISPOSITIF DE PATCH-CLAMP

(30) Priority: 09.08.2016 US 201615232770
(43) Date of publication of application: 31.10.2018
(73) Proprietor: Georgia Tech Research Corporation, Atlanta, GA 30332 (US)
(72) Inventor: KOLB, Ilya, Atlanta, Georgia 30332 (US); STOY, William, Atlanta, Georgia 30332 (US); ROUSSEAU, Erin, Clifton Park, New York 12065 (US); FOREST, Craig R., Atlanta, Georgia 30332 (US)
(74) Representative: Griebling, Onno
(86) International application number: PCT/US2016/049460
(87) International publication number: WO 2017/027888

(56) References cited:
- US-A1- 2003 022 268
- KOLB ILYA ET AL: "Cleaning Patch Clamp Pipettes Enables their Reuse", BIOPHYSICAL JOURNAL, vol. 110, no. 3, Suppl. 1, 16 February 2016 (2016-02-16), page 149A, XP029425832,
- CAROL J MILLIGAN ET AL: "Robotic multiwell planar patch-clamp for native and primary mammalian cells", NATURE PROTOCOLS, vol. 4, no. 2, 5 February 2009 (2009-02-05), pages 244-255, XP055567318, GB ISSN: 1754-2189, DOI: 10.1038/nprot.2008.230
- KAO ET AL.: 'A New Technique for Multiple Re-use of Planar Patch Clamp Chips' J NEUROSCI METHODS. vol. 208, no. 2, 15 July 2012, pages 205 - 210, XP028424850
- MALBOUBI ET AL.: 'Gigaseal Formation in Patch Clamping, Chapter 2, Development of Patch Clamping' SPRINGERBRIEFS IN APPLIED SCIENCES AND TECHNOLOGY 2014, pages 3 - 16, XP009509265
- BRUGGEMANN ET AL.: 'Microchip Technology for Automated and Parallel Patch-Clamp Recording' SMALL. vol. 2, no. 7, 24 May 2006, pages 840 - 846, XP055363300

## Description

### BACKGROUND OF THE INVENTION

### 1. Field of the Invention

The present invention relates to pipette cleaning systems and methods, and more particularly to systems and methods of cleaning patch-clamp electrodes.

### 2. Description of Related Art

Patch-clamp recording is a gold-standard single-cell electrophysiology technique that has been widely used to discover foundational biophysical properties of excitable cells. In neuroscience, the superior sensitivity and resolution of patch-clamp recording has made it an indispensable tool for discovering the tenants of ion channel activity, synaptic integration, plasticity and network connectivity in a variety of experimental preparations from cultured cells to living tissue.

Many pathological conditions such as migraine, hypertension and hypersomnia arise from dysfunctions in cell membrane-bound ion channels. Patch clamp recording is the highest resolution tool available to measure the responses of these channels to drug treatments. Patch-clamp recording provides an unprecedented ability to measure extremely small (on the order of 10⁻¹² Amperes) electrical currents arising as a result of single or multiple ion channels trafficking charged ions across the cell membrane.

There is a growing demand for large-scale single-cell measurements of electrical and molecular activity; for example for drug screening, cell type characterization, and biophysical analysis. Patch-clamp is well-suited for such studies because it can sample electrical, morphological and genetic properties of single cells and even sub-cellular compartments; however, the technique is not readily scalable.

Patch-clamp recording involves pressing a glass micropipette (namely the aperture) against a cell membrane to achieve a high-resistance (>1 GΩ) seal, or "gigaseal", with the cell membrane. These gigaseals only form when using clean micropipettes, necessitating an operator to replace the contaminated pipette with a fresh one between every contact with a cell membrane. This is a major barrier to facilitating high-throughput drug screening.

In this "conventional" approach to patch-clamp recording, a glass patch-clamp electrode (a hollow glass capillary pulled to a fine -1 µm diameter aperture at the distal end) is gently pressed against the membrane of a cell, typically under microscope guidance. A brief application of suction to the pipette forces the cell membrane to fully occlude the aperture at the distal end of the electrode. This results in the formation of an intimate physical connection between the cell membrane and the pipette aperture, termed a "gigaseal" because the resistance between the membrane-occluded pipette aperture and an extracellular ground electrode is equal to or greater than 1 gigaohm.

In both conventional and planar patch-clamp systems, to achieve a successful recording, the patch-clamp aperture must have a clean tip to form a high-resistance (≥ 1 GΩ) junction with the cell membrane. In his Nobel Prize lecture, Edwin Neher remarked that "a gigaseal could be obtained reproducibly when suction was combined with some simple measures to provide for clean surfaces, such as using a fresh pipette for each approach and using filtered solutions". However, the need for a fresh pipette for each approach or a new planar chip is a major limitation to automation and throughput In conventional systems, the ubiquitous practice of exchanging pipettes requires dexterity from the experimenter, especially when performing simultaneous patch-clamp recordings with multiple pipettes. In planar patch-clamp systems, the need to replace chips after every recording comes at a significant cost to the user.

Further, some promising studies are impractical at large scales because pipettes are not easily replaceable, for instance if they are coated, custom-manufactured, or filled with precious molecules such as synthetic peptides, novel therapeutics, human patient samples or nucleic acid constructs.

As noted above, and confirmed by Dr. Neber. it is widely accepted that cleanliness of the electrode is paramount for the formation of a gigaseal and that once an electrode has come in contact with a cell membrane, the electrode cannot be used to form a subsequent gigaseal with another cell due to adherent cellular debris. This necessitates using fresh electrodes for every patch-clamp trial in both conventional and planar patch systems.

It is thus an intention of the present invention to provide a simple, fast, and automated method for cleaning glass pipette electrodes that enables their immediate re-use. By enabling a single pipette to carry out multiple patch-clamp recordings, full automation of the technique could be achieved which would increase throughput dramatically.
*The article* 'Cleaning Patch Clamp Pipettes Enables their Reuse' by Ilya Kolb et a/ in BIOPHYSICAL JOURNAL, vol.110, no.3, suppl.1, p.149A*, states that the need to manually exchange pipettes is circumvented by an automated cleaning procedure that consists of dipping used pipettes into a commercially available detergent, pneumatically forcing the detergent into the tip, and rinsing the tip in a non-cytotoxic solution.*

### BRIEF SUMMARY OF THE INVENTION

The present invention provides a method as defined by claim 1 and a system as defined by claim 5.

The present invention provides a re-use technique applicable to the delicate electrode tip of a patch-clamp pipette. The aperture of patch-clamp electrodes are generally from 0.5-10 µm, much smaller than that of capillaries used for electrophoresis (20-200 µm), making the presently inventive methods distinct from the art of cleaning capillaries used for electrophoresis.

By immersing pipette tips into a detergent, followed by rinsing, pipettes were re-usable at least ten times with little to no degradation in signal fidelity, in experimental preparations ranging from human embryonic kidney cells to neurons in culture, slices, and *in vivo.*

In accordance with the present invention, the detergent comprises Alconox (Alconox Inc), a commercially-available detergent, at 2% w/v (20 mg/ml), for 60 seconds, which permits multiple re-uses in cell cultures, tissue slices, and *in vivo.* There was no detectable Alconox in the pipette after cleaning, and ion channel pharmacology results were indistinguishable between fresh and cleaned pipettes. The present pipette cleaning method for the first time enables dramatically improved automation of patch-clamp, as demonstrate with unattended, sequential patch-clamp recordings in cell culture and *in vivo.*

These and other objects, features and advantages of the present invention will become more apparent upon reading the following specification in conjunction with the accompanying drawing figures.

### BRIEF DESCRIPTION OF THE DRAWINGS

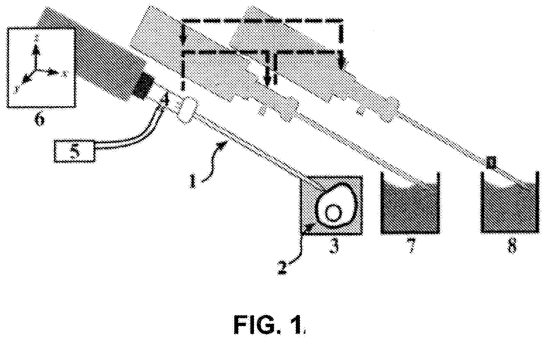

**Fig. 1** is a schematic of a conventional patch-clamp, modified to enable the present process according to an exemplary embodiment.

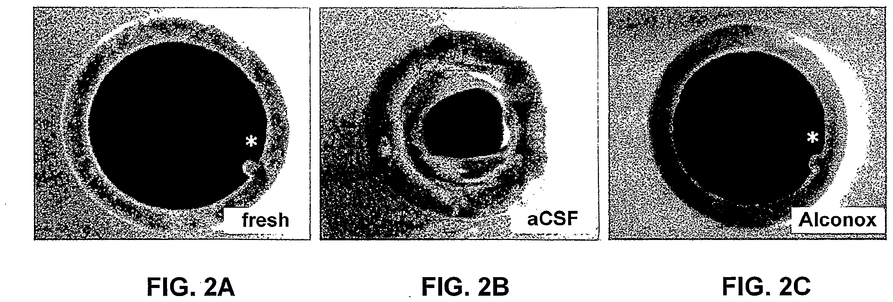

**Fig. 2A** shows a Scanning Electron Microscopy (SEM) image of a fresh pipette. **Fig. 2B** shows an SEM image of a pipette following a traditional patch-clamp trial. **Fig. 2C** shows an SEM image of a pipette following a traditional patch-clamp trial and a subsequent application of the cleaning method according to an exemplary embodiment of the present invention.

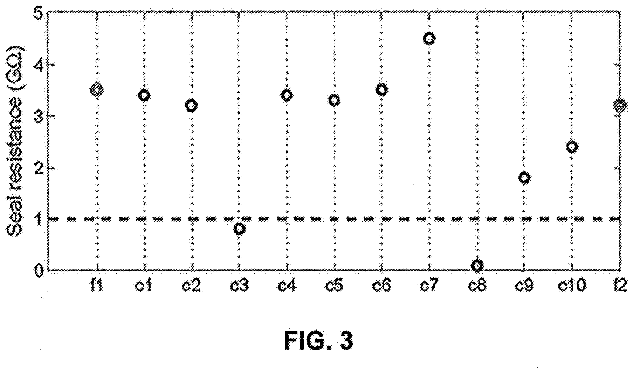

**Fig. 3** is a graph of the gigaseal resistances of ten sequential patch-clamp trials using one pipette that was cleaned according to an exemplary embodiment of the present invention.

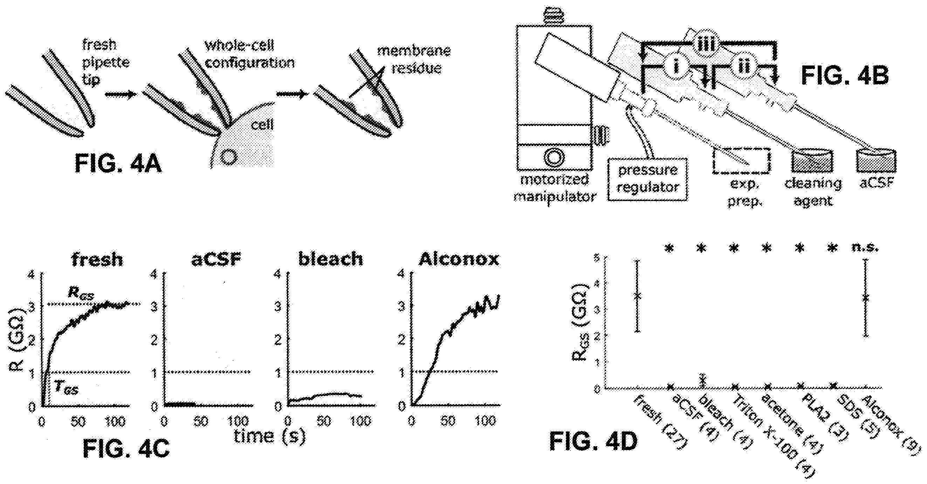

**Figs. 4****(A-D)** illustrate that during a whole-cell patch-clamp recording, cell membrane bonds to the inner walls of the pipette **(****Fig. 4A****).** A schematic of the present process according to an exemplary embodiment **(****Fig. 4B****).** Representative gigaseal formation traces **(****Fig. 4C****).** Representative resistances **(****Fig. 4D****).**

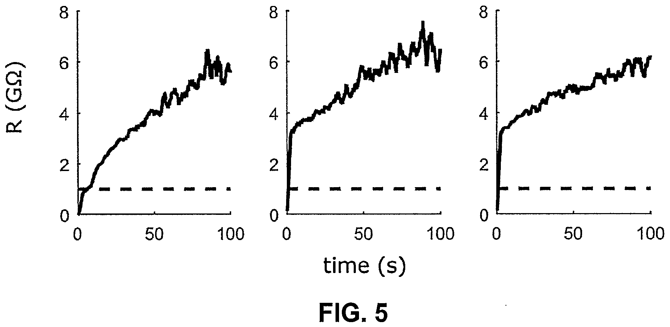

**Fig. 5** presents three representative gigaseals obtained with fresh pipettes that were pre-cleaned in Alconox. Pre-cleaning did not hinder the formation of stable gigaseals.

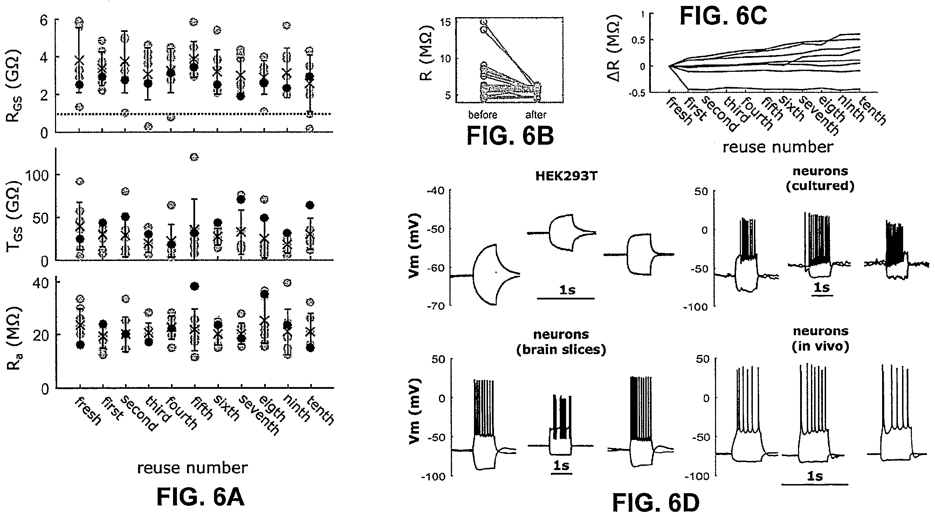

**Figs. 6****(A-D)** illustrate recording quality parameters R_{GS}, T_{GS} and Rₐ (whole-cell access resistance) **(****Fig. 6A****).** Pipette resistance before and after cleaning with Alconox **(****Fig. 6B****).** Change in pipette resistance from first to tenth re-use **(****Fig. 6C****).** Representative whole-cell responses to step current injections in different experimental preparations **(****Fig. 6D****).**

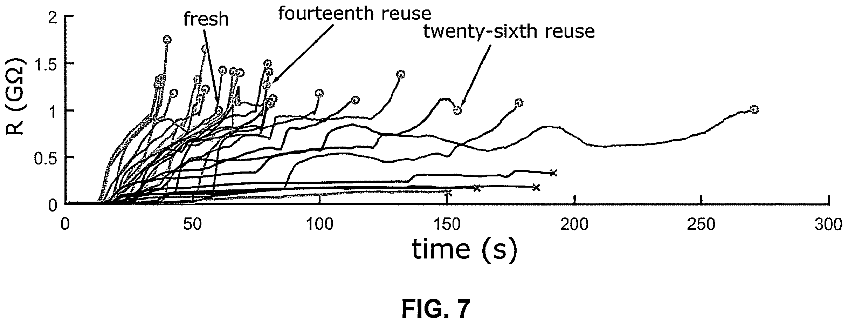

**Fig. 7** is a graph of gigaseal resistances of thirty re-use attempts with a single pipette over time.

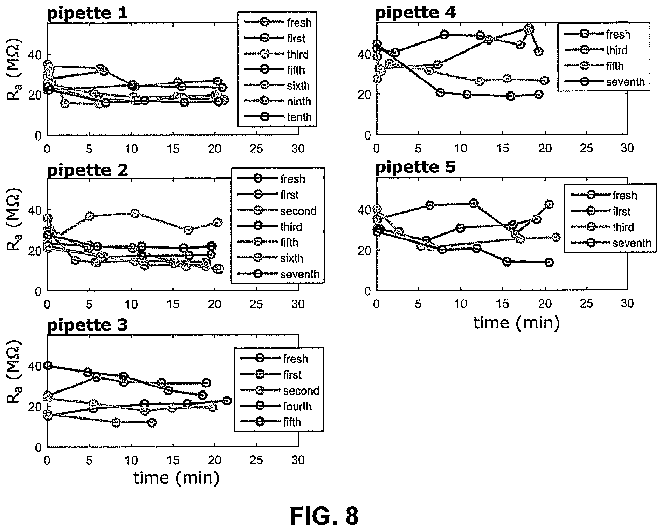

**Fig. 8** illustrates graphs for five pipettes wherein successful attempts resulting in whole-cell recordings are shown.

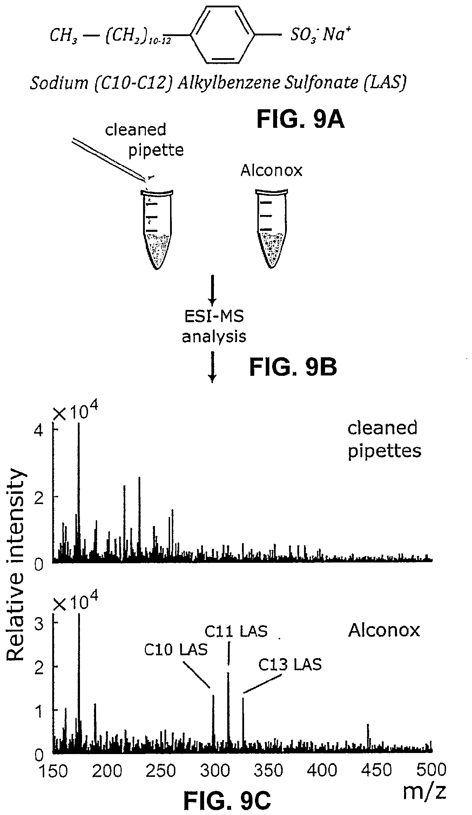

**Figs. 9****(A-C)** illustrate that LAS is the prevalent cytotoxic ingredient in Alconox. **Fig. 9A** illustrates the chemical formula for sodium (C10-C16) linear alkylbenzene sulfonate (LAS). How the contents of cleaned pipettes were collected in vials **(****Fig. 9B****).** ESI-MS spectrum **(****Fig. 9C****).**

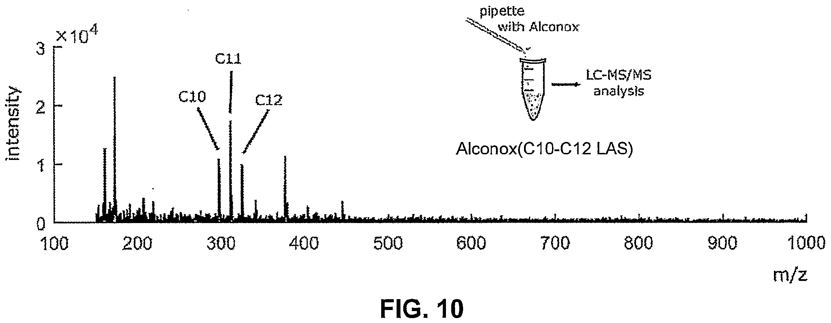

**Fig. 10** is a schematic ESI-MS spectrum characterization of pipettes containing 67 µg/ml Alconox dissolved in DI water. The main cytotoxic components of Alconox, C10-C12 LAS compounds were identified in the solution (C10: expected m/z: 297.1, found: 297.1; C11: expected m/z: 311.2, found: 311.1; C12: expected m/z: 325.2, found: 325.2).

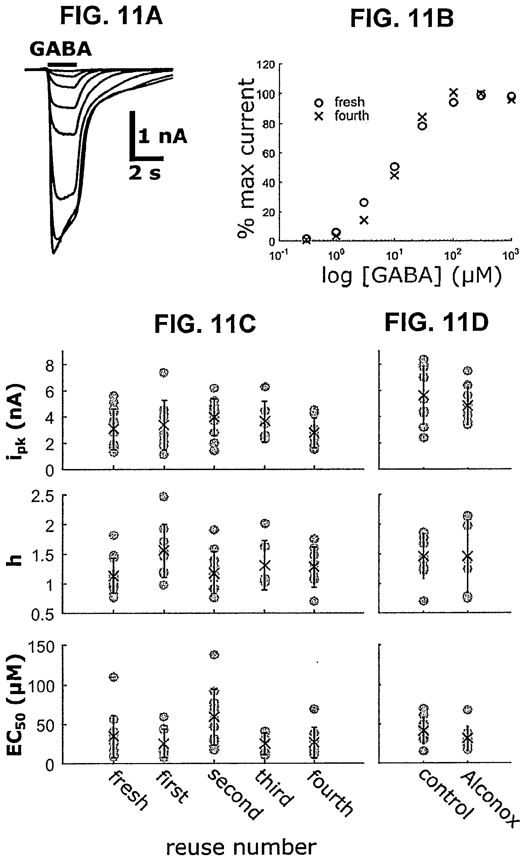

**Figs. 11****(A-D)** illustrate pipette cleaning in conjunction with GABA_{A}R pharmacology. Representative current traces recorded by whole-cell patch-clamp recording of HEK293T cells transfected with α1β2γ2s GABA_{A}Rs **(****Fig. 11A****)**. Representative normalized peak current responses to different GABA concentrations **(****Fig. 11B****).** Dose response characteristics of cells patched with fresh and re-used pipettes **(****Fig. 11C****).** That a low dose of Alconox (67 µg/mL, or equivalently, 385 × the detection limit of remaining Alconox in the pipette after cleaning) in the internal solution does not affect dose response characteristics **(****Fig. 11D****).**

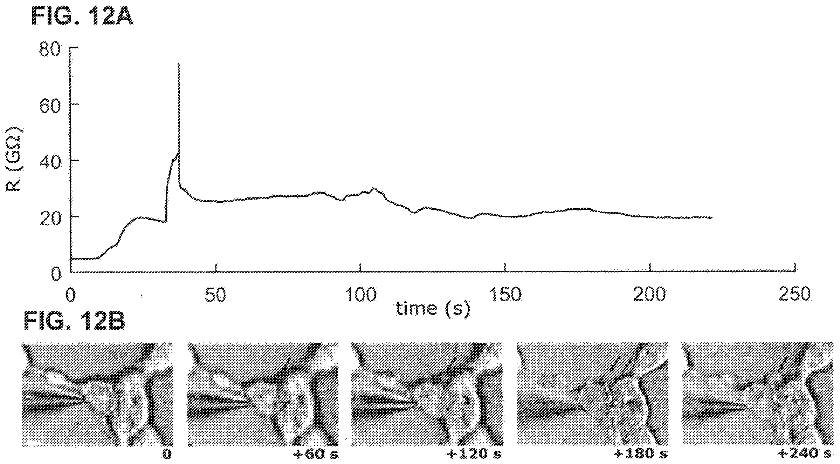

**Figs. 12****(A-B)** show a pipette containing a high dose (10 mg/mL) of Alconox damages the target cell during a patch attempt. **(****Fig. 12A****)** A gigaseal fails to form after >3 min, suggesting that Alconox is destroying cell membrane. **(****Fig. 12B****)** Cell apoptosis during the gigaseal process is evident. Arrow indicates blebs forming on the cell. Scale bar: 10 µm.

### DETAIL DESCRIPTION OF THE INVENTION

To facilitate an understanding of the principles and features of the various embodiments of the invention, various illustrative embodiments are explained below. Although exemplary embodiments of the invention are explained in detail, it is to be understood that other embodiments are contemplated. Accordingly, it is not intended that the invention is limited in its scope to the details of construction and arrangement of components set forth in the following description or illustrated in the drawings. The invention is capable of other embodiments and of being practiced or carried out in various ways. Also, in describing the exemplary embodiments, specific terminology will be resorted to for the sake of clarity.

It must also be noted that, as used in the specification and the appended claims, the singular forms "a," "an" and "the" include plural references unless the context clearly dictates otherwise. For example, reference to a component is intended also to include composition of a plurality of components. References to a composition containing "a" constituent is intended to include other constituents in addition to the one named.

Also, in describing the exemplary embodiments, terminology will be resorted to for the sake of clarity. It is intended that each term contemplates its broadest meaning as understood by those skilled in the art and includes all technical equivalents which operate in a similar manner to accomplish a similar purpose.

Ranges may be expressed herein as from "about" or "approximately" or "substantially" one particular value and/or to "about" or "approximately" or "substantially" another particular value. When such a range is expressed, other exemplary embodiments include from the one particular value and/or to the other particular value.

Similarly, as used herein, "substantially free" of something, or "substantially pure", and like characterizations, can include both being "at least substantially free" of something, or "at least substantially pure", and being "completely free" of something, or "completely pure".

By "comprising" or "containing" or "including" is meant that at least the named compound, element, particle, or method step is present in the composition or article or method, but does not exclude the presence of other compounds, materials, particles, method steps, even if the other such compounds, material, particlcs, method steps have the same function as what is named.

It is also to be understood that the mention of one or more method steps does not preclude the presence of additional method steps or intervening method steps between those steps expressly identified. Similarly, it is also to be understood that the mention of one or more components in a composition does not preclude the presence of additional components than those expressly identified.

The materials described as making up the various elements of the invention are intended to be illustrative and not restrictive. Many suitable materials that would perform the same or a similar function as the materials described herein are intended to be embraced within the scope of the invention. Such other materials not described herein can include, but are not limited to, for example, materials that are developed after the time of the development of the invention.

As shown in **Fig. 1**, in an exemplary patch-clamp embodiment of the present invention, the distal end (an apertured surface) of a glass pipette **1** is attached to a single cell **2** in a preparation area **3.** The pipette **1** is attached at the proximal end to a pipette holder **4.** A cleaning solution chamber **7** containing a cleaning solution and a rinse solution chamber **8** containing rinse solution are situated near the biological preparation area **3.** Pressure is applied via a pressure controller **5** to the pipette **1** through the pipette holder **4.** A three-axis micromanipulator **6** moves the pipette **1** and the pipette holder **4** in three dimensions.

In operation of the system of **Fig. 1**, the micromanipulator **6** is used to bring the glass pipette **1** to the preparation area **3** to approach the single cell **2.** Negative pressure is applied via the pressure controller **5** and the membrane of the cell **2** adheres to the distal end of the pipette **1.**

Following an electrophysiological experiment, the micromanipulator **6** withdraws the pipette **1** from the cell **2** and expels large, adherent cellular debris from the pipette **1** with high positive pressure from the pressure controller **5.** The micromanipulator **6** then moves the pipette **1,** under high positive pressure via the pressure controller **5,** from the preparation area **3** and into the chamber **7** containing cleaning solution. Pressure is then cycled a number of times between positive pressure and negative pressure with the pressure controller **5** to bring the cleaning solution within the chamber **7** into contact with the interior and exterior surfaces of the pipette **1** and to cyclically apply shear forces to the interior surface of the pipette **1.** The micromanipulator **6** then moves the pipette **1,** under high positive pressure from the pressure controller **5,** from the chamber **7** containing cleaning solution into the chamber **8** containing rinse solution. Pressure is then cycled a number of times between positive pressure and negative pressure with the pressure controller **5** to bring the rinse solution within the chamber **8** into contact with the interior and exterior surfaces of the pipette **1** and to remove any residual cleaning solution from the interior and exterior surfaces of the pipette **1.** The micromanipulator **6** then moves the pipette **1,** under high positive pressure from the pressure controller **5,** from the chamber containing rinse solution **8** and back to the preparation area **3.** Subsequent electrophysiological trials can be performed by repeating the steps described above.

**Figs. 2****(A-C)** are SEM images of pipette tips. **Fig. 2A** is an SEM image of a fresh pipette. Fig. **2B** shows an SEM image of a pipette tip cleaned with aCSF and being visibly contaminated with cell membrane residue, illustrating the necessity of throwing out the conventional pipette after each use (it cannot be re-used without a viable cleaning and rinsing regime heretofore unknown). **Fig. 2C** is an SEM image of a pipette following the present inventive cleaning and rinsing regime (namly using Alconox), illustrating the capability of pipette re-use. The Alconox-cleaned pipette tip resembles that of the fresh pipette. Pipette filament denoted with *. Scale bar: 1 µm.

**Fig. 3** illustrates the resulting gigaseal resistances of ten sequential patch-clamp trials using one pipette that was cleaned with the present method. In 8/10 trials, a gigaseal was successfully established.

In exemplary embodiments of the present invention shown in **Figs. 4-12****,** **Figs. 4****(A-D)** illustrate cleaning patch-clamp pipettes. During a whole-cell patch-clamp recording, cell membrane bonds to the inner walls of the pipette **(****Fig. 4A****)**). After the recording is terminated, membrane residue remains, preventing the pipette from being used for subsequent recordings. **(****Fig. 4B****)** to clean, (i) the pipette is moved from the experimental preparation to a wash bath where a cleaning agent is cycled within the tip, (ii) then to a rinse bath where the remaining cleaning agent is expelled into aCSF, (iii) and returned back to the experimental preparation. **(****Fig. 4C****)** Representative gigaseal formation traces. When using contaminated pipettes cleaned with Alconox, a multi-GΩ seal forms reliably, as would be expected when using a fresh pipette. On the other hand, cleaning with aCSF and bleach does not result in gigaseal formation. R_{GS}: maximum gigaseal resistance, T_{GS}: time (s) to reach 1 GΩ (horizontal dashed line). **(****Fig. 4D****)** Of the six tested detergents and aCSF, only Alconox reliably achieved gigaseal resistances comparable to those of fresh pipettes (p<0.001; one-way ANOVA with Dunnett's post-hoc test. *: p<0.001; n.s.: not significant, p>0.9). Data shown as mean ± s.d.; n for each cleaning agent is shown in parentheses. PLA2: Phospholipase A2; SDS: Sodium Dodecyl Sulfate.

As discussed above, during a whole-cell patch-clamp recording **(****Fig. 4A****),** cell membrane bonds to the inner walls of the pipette. After the recording is terminated, membrane residue remains, preventing the pipette from being used for subsequent recordings. After a recording attempt in an experimental preparation, pipette cleaning is accomplished in three steps **(****Fig. 4B****).** First, the pipette is moved to a bath containing a cleaning agent. Using an electronic pressure regulator, a series of alternating negative and positive pressures is applied to the pipette to cycle the cleaning agent within the tip.

Second, the pipette is moved to a bath containing aCSF. Using another series of pressure pulses, any remaining cleaning agent in the pipette is expelled. Based on the calculated fluid flow rates from pipettes (∼3 nL/s, based on pipette inner diameter of 1 µm), this pressure series is sufficient to intake the cleaning agent at least 200 µm into the lumen of the pipette. This exceeds the height of a typical lipid 'bleb' that forms in the pipette tip during a gigaseal (30-60 µm).

Lastly, the pipette is moved back to the experimental preparation. Pipettes can then be immediately re-used for a subsequent patch-clamp attempt. Refilling a pipette with internal solution is unnecessary because pipettes are commonly filled with enough solution for hundreds of attempts. All moving and cleaning steps together require 60 seconds.

**TABLE 1**

| **Cleaning solution** | **Source** |
|---|---|
| Bleach (8.25% Sodium hypochlorite) | The Clorox Company |
| Triton X-100 | X100-5mL (Sigma-Aldrich) |
| Acetone | BDH1101 (VWR) |
| Phosopolipase A₂ (PLA, 0.0001 % w/v in 10 mM HEPES buffer) | P7778 (Sigma-Aldrich) |
| Sodium Dodecylbenzenesulfonate (SDS, 1% w/v) | TCD0990 (VWR) |
| Alconox (2% w/v) | Alconox Inc. |

In accordance with the present invention, Alconox is the cleaning agent that enabled pipettes to be re-used in quantities sought. Whole-cell patch-clamp recordings on human embryonic kidney (HEK293T) cells were performed using fresh (previously unused) pipettes, cleaned them with one of six different commonly available glassware cleaning agents as shown in **TABLE 1,** as well as a control solution (aCSF), and attempted to patch another cell with the same pipette. On the second attempt, pipettes cleaned with Alconox, but not with any other cleaning agent, produced gigaseal resistances (R_{GS}) that were not significantly different from those produced by fresh pipettes **(****Figs. 4(C)(D)**). Successful whole-cell recording rates were not different between fresh and Alconox-cleaned pipettes (fresh = 92.6%, Alconox-cleaned = 88.9%, p=0.87, Fisher's exact test; other detergents and aCSF = 0%).

Gigaseals were held for 1-2 minutes to ensure stability. No gigaseals were spontaneously lost during this time in either fresh or Alconox-cleaned pipettes. When using Alconox-cleaned pipettes, an outside-out patch reliably formed after withdrawing the pipette from the cell, as would be expected if using fresh pipettes. Further, pre-cleaning fresh pipettes in Alconox had no effect on gigaseal formation. **Fig. 5** presents three representative gigaseals obtained with fresh pipettes that were pre-cleaned in Alconox. Pre-cleaning did not hinder the formation of stable gigaseals.

Using SEM, the tip of the pipettes was confirmed as being cleaned using Alconox but not aCSF **(****Figs. 2****(A-C)**). Unless stated otherwise, all subsequent experiments used Alconox.

**Figs. 6****(A-D)** illustrate that pipettes can be successfully re-used ten times. **(****Fig. 6A****)** Recording quality parameters R_{GS}, T_{GS} (defined in **Fig. 4C**) and Rₐ (whole-cell access resistance) do not decrease over ten re-uses of the same pipette (n=8 pipettes). Dashed line: 1 GΩ (threshold for gigaseal). Gray circles: individual trials; black circles: representative experiment with a single pipette. Data shown as mean ± s.d. **(****Fig. 6B****)** Pipette resistance before and after cleaning with Alconox (n=88 pairs, 8 pipettes). **(****Fig. 6C****)** Change in pipette resistance from first to tenth re-use. After ten re-uses, resistance changed (ΔR) by median: 0.175 MΩ, max: 0.61 MΩ, min: -0.44 MΩ from fresh pipette. **(****Fig. 6D****)** Representative whole-cell responses to step current injections in different experimental preparations. Recordings from HEK293T cells were obtained from the representative experiment (black circles) in **(****Fig. 6A****).** In each set, a single pipette is used for all three whole-cell recordings. In all preparations pipettes were re-used up to ten times.

As discussed above, pipettes could be re-used ten times consecutively with little to no degradation in recording quality if they were cleaned between each patch-clamp attempt **(****Fig. 6A****).** 84 HEK293T cells were successfully patched (of n=88 attempts including one fresh and 10 ten; success rate = 95%) using eight pipettes, and found no effect of the number of re-uses on gigaseal resistance (R_{GS}), the time to reach 1 GΩ (T_{GS}), and the access resistance of the patched cell (Rₐ) for each trial (one-way repeated-measures ANOVA with number of re-uses (1-10) as predictor; R_{GS}: F_{10,40}=0.99, p=0.46, n=8; T_{GS}: F_{10,40}=0.36, p=0.96, n=5, Rₐ: F_{10,40}=0.72, p=0.70, n=5).

Notably, even when pipettes failed to reach a gigaseal (e.g., R_{GS} < 1 GΩ on third, fourth re-use in **Fig. 6A**), they were successfully cleaned and re-used again, suggesting that gigaseal failures did not irreversibly contaminate the pipette tip. Beyond ten re-uses were not tested to perform a systematic study of pipette longevity; however, a pilot experiment confirmed the suspicion that pipettes could not be re-used indefinitely, when three consecutive failed patch-clamp attempts occurred after 26 re-uses.

**Fig. 7** illustrates the gigaseal resistances of thirty re-use attempts with a single pipette. The pipette was successfully re-used twenty-six times (green circles: R > 1 GΩ). The pipette did not reach a gigaseal on the fifth re-use, and consecutively on twenty-seventh through thirtieth re-use (circles: R < 1 GΩ). Gigaseal attempts are coded from light to dark according to the number of times the pipette was used. After approximately fourteen re-uses, the time to form a gigaseal increases.

The patency of the pipette tip is commonly assessed during patch-clamp recording by measuring its electrical resistance. To assess whether cleaning reduced the amount of contamination on the pipette tip, the resistance was measured twice: once immediately after completing a whole-cell recording (before cleaning) and once more after cleaning. As expected, pipette resistance before cleaning was higher than after (**Fig. 6B****;** before: median = 5.93 MΩ, range: 4.49 to 15.0 MΩ; after: median = 5.77 MΩ, range = 4.46 to 6.28 MΩ; p<0.05, Wilcoxon signed-rank test). Over 10 re-uses, the resistance of the pipette did not change significantly **(****Fig. 6C****);** p≥0.19 for first-tenth re-use, n=8, Wilcoxon signed-rank test). Together, these results suggest that cleaning eliminates residue obstructions on the pipette tip over ten re-uses.

Repeated patch-clamping in neuron cultures, acute brain slices, and *in vivo* were done using a single pipette for each experimental preparation **(****Fig. 6D****).** Cleaning did not visibly alter action potential generation in these experiments. In brain slices, successful gigaseals and successful whole-cell recordings were attained at similar rates between fresh and cleaned pipettes (gigaseals: fresh=78%, cleaned=82%, p=0.73; whole-cell recordings: fresh=39%, cleaned=48%, p=0.58, Fisher's exact test).

As shown in **Fig. 8****,** in slices, neurons were recorded and found to have the access resistance stable across that time, indicating that whole-cell recordings obtained using cleaned pipettes are stable over the duration of a typical experiment. For each plot, a single pipette was used. Only successful attempts (resulting in whole-cell recordings) are shown. Cells were held for ∼20 minutes. Access resistance did not increase for fresh pipettes as well as for pipettes re-used 1-10 times, indicating stable whole-cell recordings. *In vivo,* the Autopatcher was used to patch-clamp repeatedly with the same pipette in mouse barrel cortex (depths: 400-600 um).

### Verifying Detergent Removal After Pipette Cleaning

A major concern of using any detergent to clean pipettes is that residual surfactants in the pipette could damage the cell or affect its normal biophysical activity during a cell-attached or whole-cell patch-clamp recording. For example, Alconox comprises 33-43% sodium bicarbonate, 10-20% sodium (C10-C16) LAS, 5-15% sodium tripolyphosphate, 5-15% tetrasodium pyrophosphate, 1-10% sodium carbonate, and 1-5% sodium alcohol sulfate. LAS was chosen, a commonly-used surfactant, as a proxy for measuring Alconox concentration remaining after rinsing.

**Figs. 9****(A-C)** illustrate detection of LAS in cleaned pipettes using electrospray ionization mass spectrometry (ESI-MS). **(****Fig. 9A****)** LAS is the prevalent cytotoxic ingredient in Alconox. Alconox is composed of LAS with carbon chains lengths 10-12 (C10-C12). **(****Fig. 9B****)** The contents of cleaned pipettes were collected in vials. Known amounts of Alconox were analyzed to find the detection limit. **(****Fig. 9C****)** ESI-MS spectrum. C10-C12 LAS is not detectable in cleaned pipettes (top) but is detected in a defined Alconox solution (174 ng/mL Alconox in DI water, bottom), indicating that less than 174 ng/mL of Alconox remains in the pipettes after cleaning. C10: expected m/z: 297.1, found: 297.3; C11: expected m/z: 311.2, found: 311.2; C12: expected m/z: 325.2, found: 325.1.

As discussed above, the amount of Alconox remaining after cleaning was measured using electrospray ionization mass spectrometry (ESI-MS). As shown in **Fig. 10****,** only C10-C12 LAS was found in significant quantities in the Alconox solution, so the analysis was focused on this family of compounds **(****Fig. 9A****).** No traces of LAS was found in cleaned pipettes (**Fig. 9(B), (C)** (top)), while the instrumentation detection limit was found to be 174 ng/mL (**Fig. 9C** (bottom)). Thus, less than 174 ng/mL of Alconox remained in the pipettes after the cleaning procedure. As a control, as shown in **Fig. 10****,** LAS was ensured it could still be detected using ESI-MS when purposefully introduced in small concentrations into the pipette.

Any effect of trace amounts of Alconox in cleaned pipettes on cell receptor pharmacology would be highly undesirable. Residual LAS could disrupt gigaseal formation, thus decreasing signal quality or, more subtly, interact with amphipathic allosteric modulatory pockets on receptors, thus covertly compromising pharmacological experiments. The γ-aminobutyric acid type A Receptor (GABA_{A}R) is highly sensitive to *extracellular* application of surfactants, including LAS; its *intracellular* effects have not been thoroughly studied. Nevertheless, it is reasoned that GABA_{A}R could serve as an indicator of adverse effects of Alconox.

**Figs. 11****(A-D)** illustrate pipette cleaning in conjunction with GABA_{A}R pharmacology. **(****Fig. 11A****)** Representative current traces recorded by whole-cell patch-clamp recording of HEK293T cells transfected with α1β2γ2s GABA_{A}Rs. Black bar denotes GABA application. **(****Fig. 11B****)** Representative normalized peak current responses to different GABA concentrations. The response captured with a fresh pipette is similar to that captured with a used pipette (fourth re-use). **(****Fig. 11C****)** Dose response characteristics of cells patched with fresh and re-used pipettes. iₚₖ: peak evoked current; h: Hill coefficient; EC₅₀: half-maximal response concentration. No change in the three characteristics is observed over four re-uses. **(****Fig. 11D****)** A low dose of Alconox (67 µg/mL, or equivalently, 385 × the detection limit of remaining Alconox in the pipette after cleaning) in the internal solution does not affect dose response characteristics.

As discussed above, HEK293T cells expressing GABA_{A}R were used as a model system to verify that cleaned pipettes are pharmacologically inert. Patch-clamping using fresh and cleaned pipettes was performed, and fit the measured whole-cell current responses to increasing concentrations of GABA **(****Fig. 11A****)** to the Hill equation. Neither the peak evoked current (Iₚₖ), the Hill's coefficient (h), nor the half maximal effective concentration (EC₅₀) metrics changed as a function of re-uses (linear regression model, H₀: slope ≠ 0; Iₚₖ: p=0.998, 95% CI: -318 to 317 pA; h: p=0.719, 95% CI: -0.065 to 0.093; EC₅₀: p=0.751, 95% CI: -6.75 to 4.90).

This finding demonstrates that GABA concentration-response curves of the patch-clamped cells do not change on a population level as a function of the number of times a pipette has been re-used **(****Figs. 11(B), (C)****).** Overall, pharmacology results obtained using fresh pipettes were indistinguishable from those obtained using cleaned pipettes, suggesting no effect of trace amounts of Alconox on normal receptor function of GABA_{A}Rs.

It was also found that even if small amounts of Alconox remain in the pipette after cleaning, they do not impact gigaseal formation or GABA_{A}R pharmacology. When 67 µg/mL of Alconox (385× the ESI-MS detection limit) was intentionally dissolved in the pipette internal solution, gigaseals were still reliably achieved in all tested pipettes (n=9). No pharmacological difference between these pipettes and fresh ones was detected (**Fig. 11D****;** Iₚₖ: p=0.37; h: p=0.99; EC₅₀: p=0.19). On the other hand, when a much larger dose, 10 mg/mL Alconox (0.5 × the concentration in the wash bath) was added to the pipette, gigaseals did not form and the targeted cell exhibited clear apoptotic blebbing. See, **Figs. 12****(A-B).**

### Unattended, Sequential Patch-Clamp Recording

Previous efforts have partially or fully automated the process of obtaining a single whole-cell recording *in vitro* as well as *in vivo;* however, in all of these studies, a trained user was needed to exchange pipettes to start another attempt. Removing this manual step would enable unattended patch-clamp experiments and improve scalability and throughput. To explore this, the pipette cleaning algorithm was integrated into the previously-developed Autopatcher software. The resulting robot was tested, which was called 'patcherBot' by first performing patch-clamp recordings in HEK293T cells.

After the user selected candidate cells for patch-clamp recording, the patcherBot obtained successful gigaseals in 9/10 attempts, and successful whole-cell recordings in 6/10 attempts over the span of 33 minutes of unattended operation using a single pipette. The patcherBot was also deployed to perform blind, *in vivo* patch-clamp recordings in the mouse barrel cortex. Using four pipettes, the robot obtained successful gigaseals in 13/34 attempts, and successful whole-cell recordings in 10/34 attempts over a total span of 171 minutes *in vivo* **(****Fig. 6D****).**

### Discussion

Discovering that pipettes could be reliably cleaned and re-used multiple times was surprising given the dogmatic, decades-long practice of replacing pipettes after each patch-clamp attempt. The present simple, fast, and automated procedure comprises dipping pipettes into a commercially-available detergent, Alconox, followed by rinsing in aCSF. It is herein demonstrated the effectiveness of this cleaning method by re-using pipettes ten times, with no decrease in whole-cell recording quality in cultured cells, acute brain slices, and *in vivo.* After cleaning, the residual Alconox mass concentration in the pipettes was quantified to be less than 174 ng/mL, and shown to have no pharmacological effect on GABA_{A}Rs. Since pipette cleaning is automatic, it was integrated into the Autopatcher to perform unattended, sequential patch-clamp recordings on HEK293T cells *in vitro* and neurons *in vivo.*

Alconox is composed of a surfactant, emulsifier and water softener, and in solution, it interacts with cell membranes bound to the glass pipette tip. These three ingredients solubilize adhered lipids, stabilize the lipid micelles in solution, and enhance surfactant effectiveness, respectively. After testing various cleaning agents, it was not evident why only Alconox sufficiently cleaned pipettes to enable their re-use. Interestingly, prior studies used bleach to successfully clean planar borosilicate patch-clamp chips up to five times for whole-cell recording; yet in the present experiments using conventional pipettes, bleach did not work, suggesting that the geometry of the experimental preparation may influence cleaning effectiveness.

Overall, the precise mechanism of how cell membranes bond with glass during a gigaseal is still not understood despite notable efforts, making it difficult to devise a cleaning strategy from basic principles. A detailed biochemical study of gigaseal formation could therefore greatly inform efforts to optimize cleaning, and potentially increase the number of re-uses.

While it has herein been demonstrated that cleaning does not affect the ion channel pharmacology of GABA_{A}Rs, it was not exhaustively tested that the method in cells expressing other proteins. While several studies have characterized the effects of extracellularly applied detergents on various receptors, the intracellular effect of LAS in small concentrations is not well-understood. Thus to verify general applicability, the method should be validated using more channels, receptors, and cell types.

For many patch-clamp experiments, pipette internal solution contains ingredients that degrade over time if not refrigerated (e.g. Adenosine 5'-triphosphate magnesium salt, Guanosine 5'-triphosphate sodium salt hydrate, phosphocreatine); thus, it is expected that pipettes after multiple cleanings will have reduced concentrations of these ingredients. It is hypothesized that maintaining a chilled (i.e., 4°C) environment for pipette and internal solution, or devising a method for replacing internal solution after every trial could mitigate this deleterious effect.

The cleaning process is typically faster than manually swapping pipettes (i.e., one minute versus approximately two minutes) and does not depend on operator experience, dexterity, or fatigue level. It requires no complex hardware additions to existing electrophysiology setups and no expensive or caustic reagents. Therefore, it can be readily integrated into different experimental preparations and coupled with existing techniques that complement patch-clamp such as extracellular stimulation, two-photon microscopy, and optogenetics.

Pipette cleaning also facilitates experiments requiring specialized pipette tips. Various tip polishing techniques (using heat, pressure, or focused ion beam) and coatings can improve the ability to obtain recordings and their quality; however, these techniques are typically too time-consuming to be routine. The ability to re-use these custom-shaped pipettes could make these involved techniques more practical and scalable, reducing the need for automated pipette fabrication and inspection.

Further, the need for trained users to manually exchange pipettes is still a barrier to large-scale patch-clamp studies in the brain. In creating the patcherBot, the first robot to successfully patch-clamp multiple cells without human intervention was demonstrated. In addition to automation, cleaning could also greatly facilitate simultaneous multi-patch (i.e. dual, quadruple, octuple, etc.) experiments that have been instrumental in elucidating single-cell connectivity patterns in the brain. Since cleaning is faster than manual pipette exchange and requires no human intervention, it could increase the number and duration of simultaneous recordings.

## Claims

1. A method of detecting one or more characteristics of cells, the method comprising:
a) a first step of, with an apertured surface of a patch-clamp electrode (1), forming a resistance seal of ≥ 1 GO with a membrane of a cell (2) located in a preparation area (3), wherein the patch-clamp electrode is a pipette (1) and the apertured surface comprises the aperture of the pipette;
b) after the first step, a second step of effectively removing cellular debris from said apertured surface of said patch-clamp electrode (1) such as to enable re-use of the electrode, wherein the second step comprises a cleaning step and a rinsing step;
c) after the second step, a third step of, with said apertured surface of said patch-clamp electrode (1), forming a resistance seal of ≥ 1 GΩ with a membrane of a different cell (2) located in said preparation area (3);
**characterized**
**in that** the cleaning step comprises the steps of:
- applying high pressure to the pipette (1) to expel large adherent cellular debris from the electrode,
- moving the pipette (1) into a chamber (7) containing a cleaning solution, and
- cycling pressure a number of times between positive pressure and negative pressure to bring the cleaning solution into contact with the interior and exterior surfaces of the pipette (1) and to cyclically apply shear forces to the interior surface of the pipette (1);
**in that** the rinsing step comprises the steps of:
- moving the pipette (1) into a chamber (8) containing a rinse solution, and
- cycling pressure a number of times between positive pressure and negative pressure to bring the rinse solution into contact with the interior and exterior surfaces of the pipette (1) and to remove any residual cleaning solution from the interior and exterior surfaces of the pipette (1); and
**in that** the cleaning solution comprises 33-43% sodium bicarbonate, 10-20% sodium (C10-C20) LAS, 5-15% sodium tripolyphosphate, 5-15% tetrasodium pyrophosphate, 1-10% sodium carbonate, and 1-5% sodium alcohol sulfate.

2. Method according to claim 1, wherein the cleaning step is via agency of an automated process (6).

3. Method according to claim 1 or 2, wherein the cleaning step occurs in 1 minute.

4. Method according to any of claims 1-3, wherein the second step enables re-use of at least portions of the same apertured surface with at least five different cells.

5. A system for detecting one or more characteristics of cells, the system comprising a patch-clamp electrode (1) having an apertured surface, wherein the
patch-clamp electrode is a pipette (1) and the apertured surface comprises the aperture of the pipette;
a preparation area (3) adapted for said patch-clamp electrode (1) to form a resistance seal with a membrane of a cell (2);
a suction assembly in communication with the apertured surface and adapted to provide each resistance seal with a resistance ≥ 1 GΩ;
a re-use assembly;
**characterized in that** the system is adapted to perform the method of any of claims 1-4, wherein the re-use assembly:
- comprises a cleaning solution chamber (7) situated near the preparation area (3) and containing a cleaning solution that comprises 33-43% sodium bicarbonate, 10-20% sodium (C10-C20) LAS, 5-15% sodium tripolyphosphate, 5-15% tetrasodium pyrophosphate, 1-10% sodium carbonate, and 1-5% sodium alcohol sulfate;
- comprises a rinse solution chamber (8) situated near the preparation area (3) and configured to contain rinse solution;
- comprises a pressure assembly in communication with the apertured surface and adapted to cyclically provide alternating positive and negative pressures of up to ∓1,500 mbar relative to atmosphere, to bring the cleaning solution and the rinse solution into contact with the interior and exterior surfaces of the pipette (1); and
- is adapted to clean at least portions of the same apertured surface so that it can be re-used to form a resistance seal with membranes of at least five different cells, preferably with membranes of at least seven different cells.

6. The system of claim 5, wherein the rinse solution chamber (8) contains the rinse solution.

## Patentansprüche

1. Verfahren zum Nachweis einer oder mehrerer Eigenschaften von Zellen, wobei das Verfahren umfasst:
a) einen ersten Schritt, bei dem mit einer offenen Oberfläche einer Patch-Clamp-Elektrode (1) eine Widerstandsdichtung von ≥ 1 GΩ mit einer Membran einer Zelle (2) geformt wird, die sich in einem Präparationsbereich (3) befindet, wobei die Patch-Clamp-Elektrode eine Pipette (1) ist und die offene Oberfläche die Öffnung der Pipette umfasst;
b) nach dem ersten Schritt einen zweiten Schritt des effektiven Entfernens von Zellschmutz von der offenen Oberfläche der Patch-Clamp-Elektrode (1), um eine Wiederverwendung der Elektrode zu ermöglichen, wobei der zweite Schritt einen Reinigungsschritt und einen Spülschritt umfasst;
c) nach dem zweiten Schritt einen dritten Schritt, bei dem mit der offenen Oberfläche der Patch-Clamp-Elektrode (1) eine Widerstandsdichtung von ≥ 1 GΩ mit einer Membran einer anderen Zelle (2) gebildet wird, die sich in dem Präparationsbereich (3) befindet;
**dadurch gekennzeichnet, dass** der Reinigungsschritt die folgenden Schritte umfasst:
- Anwendung von hohem Druck auf die Pipette (1), um großen anhaftenden Zellschmutz von der Elektrode zu vertreiben,
- Bewegen der Pipette (1) in eine Kammer (7), die eine Reinigungslösung enthält, und
- mehrmaliges Wechseln zwischen Überdruck und Unterdruck, um die Reinigungslösung mit der inneren und äußeren Oberfläche der Pipette (1) in Kontakt zu bringen und zyklisch Scherkräfte auf die Innenfläche der Pipette (1) auszuüben;
dadurch, dass der Spülschritt die folgenden Schritte umfasst:
- Bewegen der Pipette (1) in eine Kammer (8), die eine Spüllösung enthält, und
- mehrmaliges Wechseln zwischen Überdruck und Unterdruck, um die Spüllösung mit der inneren und äußeren Oberfläche der Pipette (1) in Kontakt zu bringen und irgendwelche Reste der Reinigungslösung von der inneren und äußeren Oberfläche der Pipette (1) zu entfernen; und
dass die Reinigungslösung 33-43% Natriumbicarbonat, 10-20% Natrium (C10-C20) LAS, 5-15% Natriumtripolyphosphat, 5-15% Tetranatriumpyrophosphat, 1-10% Natriumcarbonat und 1-5% Natriumalkoholsulfat enthält.

2. Verfahren nach Anspruch 1, wobei der Reinigungsschritt durch Vermittlung eines automatisierten Prozesses (6) erfolgt.

3. Verfahren nach Anspruch 1 oder 2, wobei der Reinigungsschritt in 1 Minute erfolgt.

4. Verfahren nach einem der Ansprüche 1 bis 3, wobei der zweite Schritt die Wiederverwendung zumindest von Teilen der gleichen offenen Oberfläche mit mindestens fünf verschiedenen Zellen ermöglicht.

5. System zum Erfassen einer oder mehrerer Eigenschaften von Zellen, wobei das System eine Patch-Clamp-Elektrode (1) mit einer offenen Oberfläche umfasst, wobei die Patch-Clamp-Elektrode eine Pipette (1) ist und die offene Oberfläche die Öffnung der Pipette umfasst;
einen Präparationsbereich (3), der für die Patch-Clamp-Elektrode (1) geeignet ist, um eine Widerstandsdichtung mit einer Membran einer Zelle (2) zu bilden;
eine Ansaugvorrichtung, die mit der offenen Oberfläche in Verbindung steht und so ausgelegt ist, dass sie jede Widerstandsdichtung mit einem Widerstand ≥ 1 GΩ versieht;
eine wiederverwendbare Baugruppe;
**dadurch gekennzeichnet, dass** das System angepasst ist, um das Verfahren nach einem der Ansprüche 1-4 durchzuführen, wobei die wiederverwendbare Baugruppe:
- eine Reinigungslösungskammer (7) umfasst, die sich in der Nähe des Präperationsbereichs (3) befindet und eine Reinigungslösung enthält, die 33-43% Natriumbicarbonat, 10-20% Natrium-(C10-C20)-LAS, 5-15% Natriumtripolyphosphat, 5-15% Tetranatriumpyrophosphat, 1-10% Natriumcarbonat und 1-5% Natriumalkoholsulfat umfasst;
- eine Spüllösungskammer (8) umfasst, die sich in der Nähe des Präperationsbereichs (3) befindet und so eingerichtet ist, dass sie Spüllösung enthält;
- eine Druckbaugruppe umfasst, die mit der offenen Oberfläche in Verbindung steht und geeignet ist, zyklisch abwechselnd positive und negative Drücke von bis zu ∓ 1.500 mbar relativ zur Atmosphäre zu erzeugen, um die Reinigungslösung und die Spüllösung mit den inneren und äußeren Oberflächen der Pipette (1) in Kontakt zu bringen; und
- geeignet ist, zumindest Teile der offenen Oberfläche zu reinigen, so dass sie wiederverwendet werden kann, um eine Widerstandsdichtung mit Membranen von mindestens fünf verschiedenen Zellen, vorzugsweise mit Membranen von mindestens sieben verschiedenen Zellen, zu bilden.

6. Das System nach Anspruch 5, wobei die Spüllösungskammer (8) die Spüllösung enthält.

## Revendications

1. Procédé de détection d'une ou plusieurs caractéristique(s) de cellules, lequel procédé comporte :
a) une première étape au cours de laquelle, avec une surface d'ouverture d'une électrode patch-clamp (1), on forme une jonction étanche, de résistance supérieure ou égale à 1 GΩ avec une membrane d'une cellule (2) placée dans une zone de préparation (3), étant entendu que l'électrode patch-clamp est une pipette (1) et que la surface d'ouverture comprend l'orifice de la pipette,
b) après cette première étape, une deuxième étape au cours de laquelle on ôte efficacement les débris de cellule de ladite surface d'ouverture de ladite électrode patch-clamp (1) de manière à pouvoir réutiliser cette électrode, laquelle deuxième étape comporte une opération de nettoyage et une opération de rinçage,
c) et après cette deuxième étape, une troisième étape au cours de laquelle, avec ladite surface d'ouverture de ladite électrode patch-clamp (1), on forme une jonction étanche, de résistance supérieure ou égale à 1 GΩ, avec une membrane d'une cellule (2) différente, placée dans ladite zone de préparation (3),
et est **caractérisé**
**en ce que** l'opération de nettoyage comporte les étapes suivantes :
- appliquer une haute pression à la pipette (1), pour expulser de l'électrode les gros débris de cellule qui y adhèrent,
- placer la pipette (1) dans une enceinte (7) contenant une solution de nettoyage,
- et faire varier la pression en cycles, un certain nombre de fois, entre une pression positive et une pression négative, afin de mettre la solution de nettoyage en contact avec les surfaces interne et externe de la pipette (1) et d'appliquer de manière cyclique des forces de cisaillement sur la surface interne de la pipette (1),
**en ce que** l'opération de rinçage comporte les étapes suivantes :
- mettre la pipette (1) dans une enceinte (8) contenant une solution de rinçage,
- et faire varier la pression en cycles, un certain nombre de fois, entre une pression positive et une pression négative, afin de mettre la solution de rinçage en contact avec les surfaces interne et externe de la pipette (1) et d'éliminer tout résidu de la solution de nettoyage des surfaces interne et externe de la pipette (1),
et **en ce que** la solution de nettoyage comprend de 33 à 43 % de bicarbonate de sodium, de 10 à 20 % de (alkyle linéaire en C₁₀-C₂₀)-benzène-sulfonate de sodium, de 5 à 15 % de tripolyphosphate de sodium, de 5 à 15 % de pyrophosphate de tétrasodium, de 1 à 10 % de carbonate de sodium et de 1 à 5 % d'alcool-sulfate de sodium.

2. Procédé conforme à la revendication 1, dans lequel l'opération de nettoyage s'effectue par un processus automatisé (6).

3. Procédé conforme à la revendication 1 ou 2, dans lequel l'opération de nettoyage s'effectue en 1 minute.

4. Procédé conforme à l'une des revendications 1 à 3, dans lequel la deuxième étape permet de réutiliser au moins des parties de la même surface d'ouverture avec au moins cinq cellules différentes.

5. Système de détection d'une ou plusieurs caractéristique(s) de cellules, lequel système comporte :
- une électrode patch-clamp (1) dotée d'une surface d'ouverture, étant entendu que l'électrode patch-clamp est une pipette (1) et que la surface d'ouverture comprend l'orifice de la pipette,
- une zone de préparation (3), adaptée pour que ladite électrode patch-clamp (1) forme une jonction étanche à résistance avec une membrane d'une cellule (2),
- un appareillage de succion, en communication avec la surface d'ouverture et adapté pour conférer à chaque jonction étanche une résistance supérieure ou égale à 1 GΩ,
- et un appareillage de réutilisation,
**caractérisé en ce que** ce système est adapté pour mettre en œuvre un procédé conforme à l'une des revendications 1 à 4,
et dans lequel système l'appareillage de réutilisation :
- comprend une chambre (7) pour solution de nettoyage, située auprès de la zone de préparation (3) et contenant une solution de nettoyage qui comprend de 33 à 43 % de bicarbonate de sodium, de 10 à 20 % de (alkyle linéaire en C₁₀-C₂₀)-benzène-sulfonate de sodium, de 5 à 15 % de tripolyphosphate de sodium, de 5 à 15 % de pyrophosphate de tétrasodium, de 1 à 10 % de carbonate de sodium et de 1 à 5 % d'alcool-sulfate de sodium,
- comprend une chambre (8) pour solution de rinçage, située auprès de la zone de préparation (3) et configurée pour contenir une solution de rinçage,
- comprend un appareillage à pression, en communication avec la surface d'ouverture et adapté pour établir, de façon cyclique et en alternance, une pression positive ou négative, valant jusqu'à ± 1500 millibars par rapport à la pression atmosphérique, pour mettre la solution de nettoyage et la solution de rinçage en contact avec les surfaces interne et externe de la pipette (1),
- et est adapté pour nettoyer au moins des parties de la même surface d'ouverture de manière qu'on puisse réutiliser celle-ci pour former une jonction étanche à résistance avec les membranes d'au moins cinq cellules différentes, et de préférence, avec les membranes d'au moins sept cellules différentes.

6. Système conforme à la revendication 5, dans lequel la chambre (8) pour solution de rinçage contient la solution de rinçage.
